Europäisches Patentamt

European Patent Office

Office européen des brevets

(19)

(11) Publication number: **0 316 677**
**A2**

(12) **EUROPEAN PATENT APPLICATION**

(21) Application number: **88118362.8**

(22) Date of filing: **04.11.88**

(51) Int. Cl.⁴: **C12N 15/00 , C12N 1/20 ,**
**//(C12N1/20,C12R1:46)**

(30) Priority: **13.11.87 US 120385**

(43) Date of publication of application:
**24.05.89 Bulletin 89/21**

(84) Designated Contracting States:
**DE FR GB IT NL**

(71) Applicant: **MILES INC.**
**1127 Myrtle Street**
**Elkhart,IN 46515(US)**

(72) Inventor: **Sanders, Mary Ellen**
**52935 Marvin Street**
**Elkhart, IN 46514(US)**
Inventor: **Shultz, John William**
**1504 Bower Street**
**Elkhart, IN 46514(US)**

(74) Representative: **Dänner, Klaus, Dr. et al**
**c/o Bayer AG Konzernverwaltung RP**
**Patentabteilung**
**D-5090 Leverkusen 1 Bayerwerk(DE)**

(54) **Method for cloning in lactic acid bacteria.**

(57) A plasmid-borne gene from a first lactic acid bacterium is cloned in a second lactic acid bacterium by means of a cloning vector that contains a gene for a phenotypic marker and that is devoid of an origin of replication for the second bacterium. The plasmid from the first lactic acid bacterium is combined with the cloning vector to produce a recombinant plasmid that is easily selectable in the second lactic acid bacterium and that contains both the gene to be cloned and an origin of replication for the second lactic acid bacterium. The recombinant plasmid or plasmid-borne functional gene segment so identified can be inserted into a lactic acid bacterial host by conjugation or transformation to provide an improved starter culture bacterial strain.

EP 0 316 677 A2

## METHOD FOR CLONING IN LACTIC ACID BACTERIA

### Field of the Invention

This invention relates to the improvement of lactic acid bacteria that are used as starter cultures in the production of fermented milk products. In particular, the invention relates to methods for cloning useful genes from a first lactic acid bacterium into a transformable second lactic acid bacterial host cell in which the genes were not previously present and for isolating the transformed bacterial host cells in which the genes have been cloned.

### Background of the Invention

Lactic acid-producing bacteria are valuable articles of commerce useful in the production of fermented milk products, such as cheese, yogurt, sour cream, buttermilk, acidophilus milk and the like. Industrially important lactic acid bacteria include, for example, S. lactis, S. cremoris, S. thermophilus, Lactobacillus ssp., including casei, delbrueckii, helveticus, bulgaricus and acidophilus, Leuconostoc ssp., Pediococcus ssp. and Propionibacterium. The cloning of industrially important genes from lactic acid bacteria is an important objective in constructing improved starter cultures for use in the production of fermented milk products.

Examples of lactic acid bacterial genes that have been cloned include enzymes EII-lac, factor-III-lac and phospho-$\beta$-galactosidase (P-$\beta$-gal) from S. lactis C2; P-$\beta$-gal and either EII-lac or FIII-lac from S. lactis 712; P-$\beta$-gal and either EII-lac or FIII-lac from S. cremoris; $\beta$-D-gal from S. thermophilus; P-$\beta$-gal fromS thermophilus; P-$\beta$-Gal fromLactobacillus casei; tagatose-1,6-biphosphate aldolase from S. lactis H1; the malolactic gene from Lactobacillus delbrueckii; the malolactic gene from Leuconostoc oenos; and two proteolytic enzymes from S. cremoris. Most work to date on cloning lactic acid genes has centered on metabolic genes for lactose utilization.

Many genes of industrial importance are encoded by plasmid DNA in lactic acid bacteria. Unfortunately, most genes of interest from lactic acid bacteria are not easily selectable. That is, they do not confer a phenotype which allows facile separation of microorganisms which contain the genes from those which do not. Moreover, unlike organisms such as E. coli and B. subtilis, lactic acid bacterial genetics are relatively poorly understood. For example complex genetic systems which confer desirable properties, such as phage resistance, have not been fully characterized. Characteristics such as the location and size of particular genes and even the number and nature of genes in some lactic acid bacterial operons are often unknown. Although the genetic structure and organization responsible for a desired trait may be unknown, there is nevertheless a need for a means to transfer such genetic traits directly from one lactic acid bacterium to another. It would be very useful to provide a simple means for cloning genes from lactic acid bacterial plasmids and for isolating host cells containing the cloned gene.

Gasson, M.J. and Anderson, P.H., described the preparation of cloning vectors by ligation of a cryptic S. lactis plasmid, pSH71, to antibiotic resistance genes from Bacillus vector, pBD64. (FEMS Microbiol. Lttr., 30, 193-196 (1985)). Selection relied on a bifunctional origin of replication derived from S. lactis and capable of expression in Bacillus. The vector was first cloned into Bacillus. The DNA from the Bacillus transformant was purified and that purified DNA was then transformed into a plasmid-free S. lactis.

Indirect cloning procedures such as those described by Gasson and Anderson, contemplate genetic manipulations and selection in B. subtilis, rather than in a lactic acid bacterium. Since many lactic acid bacterial genes are not expressed in B. subtilis, these indirect cloning procedures generally require a rather substantial understanding of the genetic structures being cloned. The cloning procedure would be unsuccessful if any of the genetic manipulations resulted in deletion or insertional inactivation of a desired gene. Such results often would not be apparent until the recombinant plasmid were isolated from the B. subtilis and introduced into the intended lactic acid bacterial host.

Genes which confer bacteriophage resistance upon lactic acid bacteria are of particular interest in the improvement of starter cultures, inasmuch as certain important lactic acid species, e.g. S. lactis, do not contain suitable native genes for phage resistance. Phage infections during milk fermentation constitute a major problem, accounting for considerable loss of product each year. An abstract on the identification and cloning of plasmid DNA encoding for reduced phage sensitivity from S. lactis subsp. diacetylactis KR2 was

EP 0 316 677 A2

distributed at the American Dairy Science Association 82nd Annual Meeting, June 21-24, 1987 at the University of Missouri-Columbia.

U.S. Patent No. 4,530,904, issued to Hershberger and Rosteck, relates to a method of protecting a bacterium in a closed fermentor from a naturally occurring bacteriophage. The patent discloses the cloning of HhaII restriction-modification system of Haemophilus haemolyticus into an E. coli host. The transformation procedures employed for E. coli would not work in lactic acid bacteria, and the plasmids described in the patent do not have origins of replication that would function in lactic acid bacteria.

## Summary of the Invention

The present invention provides a method for producing a transformed lactic acid bacterium from a lactic acid bacterial host and isolating said transformed lactic acid bacterium, wherein the transformed lactic acid bacterium contains a plasmid-borne functional gene segment capable of confering a phenotype not previously present in the host bacterium. In particular, the method of the invention includes the steps of:

(a) forming a recombinant plasmid by joining, under DNA-ligating conditions, (i) a first plasmid from a lactic acid bacterium, said plasmid having a gene of interest and an origin of replication which is functional in the lactic acid bacterial host with (ii) a vector which contains a selectable marker but does not contain an origin of replication which is functional in the lactic acid bacterial host;

(b) directly transforming the lactic acid bacterial host with the recombinant plasmid under transforming conditions;

(c) cultivating the transformants resulting from step (b) and selecting and isolating a transformant which exhibits the phenotype conferred to the cell by the selectable marker.

A vector useful for producing the transformed lactic acid bacterium is also provided. The vector includes a marker which is selectable in the host lactic acid bacterium, but is devoid of an origin of replication for the host lactic acid bacterium. Preferably the vector includes an origin of replication which is functional in a bacterial species used for constructing, producing and maintaining the vector, but which is nonfunctional in the lactic acid bacterial host.

Also provided by the present invention is a transformed lactic acid bacterial host which contains a recombinant plasmid capable of conferring a phenotype not previously present in the host bacterium.

In a preferred embodiment, the functional gene segment to be cloned is a phage resistance gene from S. cremoris and the host is S. lactis. Advantageously, the selectable marker is a gene which confers antibiotic resistance, such as, for example, an erythromycin resistance (Erm$^r$) gene. Thus, for example, S. lactis can be transformed with a recombinant plasmid containing the phage resistance gene from S. cremoris and transformants can be selected by culturing in the presence of erythromycin.

There are also provided by the present invention transformant bacteria comprising lactic acid host cells containing recombinant plasmids. The plasmids comprise a gene encoding a marker selectable in the host cell, a gene encoding a gene product that was not previously present in the host cell and an origin of replication that is functional in the host cell. The direct cloning procedure of this invention permits cloning complex and relatively uncharacterized genetic structures from one lactic acid bacterium directly to another. The procedure does not require the use of "shuttle" vectors or intermediate selections in non-lactic acid bacteria.

The plasmid-borne gene of interest thus isolated can be moved directly into a starter culture lactic acid bacterium by transformation or conjugation or the functional gene segment thereof can be isolated and inserted into a starter culture lactic acid bacteria to provide an improved strain.

## Brief Description of the Drawings

Figure 1 discloses a scheme for construction of a cloning vector deficient in an origin of replication for the lactic acid bacteria and the use of this vector to clone pMEIa. Step I: Digest pSA3 with HindIII(H), ligate fragments, transform into E. coli and select Tet$^r$ and Cm$^r$ transformants. One of these transformants harbors pSA34, which results from the deletion of a substantial portion of pSA3. Step II: linearize pMEIa by partial digestion with HindIII. Step III: linearize pSA34 by digestion with HindIII. Step IV: ligate HindIII

3

digested pMEla and pSA34. Select Erm$^r$ and screen for phage resistance. Examples of clones resulting from this ligation include pME34, pME35, pME36, pME37 (all phage-resistant) and pHB1, pHP2 and pHP4 (all phage-sensitive).

Figure 2 is an agarose gel showing the migration patterns of HindlII and Clai digests of various plasmids employed in the invention.

Figure 3 is a partial restriction map of plasmid pME34. HindIII A, B and C fragments and the position of pSA34 are labeled.

# DEFINITIONS

As used herein, the following terms shall have the assigned meanings unless a different meaning is indicated expressly or by context.

Plasmid - A plasmid is a unit of extra-chromosomal DNA which contains an origin of replication for its host.

Vector - A vector is a unit of extra-chromosomal DNA, such as a plasmid or certain bacteriophages, which is used for carrying a segment of DNA into a cell. A vector commonly contains an origin of replication and usually also contains one or more selectable markers functional in a specific bacterial host.

(ori) - An origin of replication (ori) is a region of DNA required for the replication of DNA autonomously from the chromosome. "ori$^+$" denotes the presence of such a region; "ori$^-$" denotes its absence.

Gene product - A gene product is the result of transcription and/or translation and may be protein or RNA.

pSA34 - pSA34 is a vector derived from pSA3 composed of approximately 6 kb of DNA, encoding tetracycline and chloramphenicol resistance in E. coli, erythromycin resistance in streptococci, capable of autonomous replication in E. coli but not in the streptococci.

pMEla - pMEla is a plasmid of 17.5 kb obtained from S. cremoris KH and includes a phage resistance for lactic streptococci.

Lactic acid bacteria - Lactic acid bacteria are a group of gram positive, commonly non-pathogenic, bacteria functionally related due to their importance in food fermentation. Most of these bacteria produce lactic acid from lactose or other carbohydrates; however, some bacteria included in this group such as Leuconostoc cremoris and Propionibacteria produce important flavor compounds other than lactic acid. The bacteria most commonly referred to as lactic acid bacteria include species of the genera Lactobacillus, Streptococcus, Pediococcus, Leuconostoc, and Propionibacterium.

$\phi^R/\phi^S$- A bacterium is considered phage-sensitive, $\phi^S$, if it is susceptible to phage infection and propagation. A host bacterium is considered phage-resistant, $\phi^R$, if it resists phage infection and propagation. Different degrees of phage resistance or sensitivity can occur and are often described by relative plaque counts or efficiency of plating.

Efficiency of plating (EOP) - Efficiency of plating (EOP) is a term describing the relative phage resistance or sensitivity of a pair of bacteria. EOP is defined as the titer of phage on a test strain divided by the titer of phage on a fully phage sensitive strain.

Restriction/modification (R/M) - A restriction/modification (R/M) is an enzyme system which can confer phage resistance. Restriction is characterized in vivo by a decrease in EOP relative to a non-restricting host. Modification is characterized as the restoration of full level phage plaquing after one cycle of growth through the restricting host "RIM$^+$" denotes the presence of such a system; "RIM$^-$" denotes its absence".

Selectable marker - A selectable marker is a gene or gene system encoding a phenotype that enables direct recognition from a single plating of a host bacterium possessing the gene(s).

Screenable marker - A screenable marker is a gene or gene system encoding a phenotype that requires secondary testing to recognize a host bacterium possessing the gene(s).

Transformation - Transformation is the introduction of DNA into a host cell. Transformation results in a genotypic change in the host cell. Transformation may be accomplished by protoplast and nonprotoplast methods.

Protoplast transformation - Protoplast transformation is a method of transformation which requires the deliberate removal of cell wall material from the host cell using artificially added enzyme to form protoplasts and the creation of conditions favorable for DNA uptake by the host cell.

Non-protoplast transformation - Non-protoplast transformation is a method of transformation which does not require the intentional enzymatic removal of cell wall material for transformation.

Transformant - A transformant is a bacterium that can be identified as having undergone transformation.

Plaque forming units (pfu) - Plaque forming units (pfu) are clear zones in a lawn of host indicator cells resulting from lysis by a phage particle. Phage enumerations are reported as pfu/ml.

Erm$^r$ - The erythromycin resistance phenotype.
Erm$^s$ - The erythromycin sensitive phenotype.
Tet$^r$ - The tetracycline resistant phenotype.
Tet$^s$ - The tetracycline sensitive phenotype.
Cm$^r$ - The chloramphenicol resistant phenotype.
Cm$^s$ - The chloramphenicol sensitive phenotype.
Lactic Streptococci - Lactic streptococci is a group of bacteria composed of Streptococcus lactis, Streptococcus cremoris, Streptococcus diacetylactis and Streptococcus thermophilus.
Starter culture - A starter culture is a lactic acid bacterial culture that may include single or multiple strains, species or genera used to inoculate a food substrate for fermentation. A starter culture lactic acid bacterium commonly has the ability to ferment milk or milk products such as whey.
Lac$^+$ - The abbreviation "Lac$^+$" denotes the ability of a bacterium to metabolize lactose. Commercially useful lactic acid bacteria commonly metabolize lactose to lactic acid.
Prt$^+$ - The abbreviation "Prt$^+$" denotes the ability to digest protein to provide nitrogeneous substrates for growth in milk. Commercially useful lactic acid bacteria commonly exhibit this phenotype.

Detailed Description of the Preferred Embodiment

The method of the invention is generally applicable to producing an improved strain of lactic acid bacteria which contains a recombinant plasmid having a functional gene segment capable of confering a phenotype not previously present in the strain. The method relies on the use of an ori deficient vector.

The invention provides an efficient method for identifying a plasmid-borne gene of interest conferring a phenotype which does not allow facile separation of microorganisms which contained the gene from those which do not. Once identified and isolated by the method of the invention the gene can readily be inserted into a host of commercial importance, such as a lactic acid bacterium useful as a starter culture, by transformation or conjugation. The plasmid can be inserted intact or the functional gene segment of interest can be identified and inserted.

Any gene of interest can be introduced into lactic acid bacteria by this process, provided that it is carried by a plasmid containing an ori region which is functional in the lactic acid bacterial host. Plasmid-borne lactic acid bacterial genes which can be cloned in this manner, in addition to phage resistance genes, include, for example, lactose metabolic genes, proteolytic enzymes, citrate-metabolizing enzymes, bacteriocin-producing enzymes, bacteriocin resistance, or conjugal transfer genes. Phage resistance is a particularly desirable phenotype. Such resistance may be conferred by the ability of the host to produce a restriction/modification enzyme system and by other mechanisms. It may be desirable to produce a lactic acid bacterial strain having more than one mechanism of phage resistance, such as restriction/modification, abortive infection, prevention of phage adsorption or lysogenic immunity.

The method is particularly applicable to moving a functional gene segment from one lactic acid bacterial strain to another, especially from one streptoccocal lactic acid bacterial strain to another. These strains are particularly useful in producing cheese and other fermented milk products. Within the lactic acid bacteria many desirable phenotypes are encoded by plasmid-borne genes.

A selectable marker is used to permit direct selection of the desired transformant. Preferred selectable markers include, for example, antibiotic resistance markers such as tetracycline or erythromycin resistance, kanamycin resistance or traits natural to lactic acid bacteria including bacteriocin or nisin resistance. Other selectable markers, such as antibiotic resistant markers or essential nutrient complementation markers may also be used.

The vector employed in this method preferably contains means for effecting its propagation in a host species (referred to herein as a secondary host species) different from the lactic acid bacterial host. Such means can include, for example, an origin of replication which is functional in E. coli; such as that from a plasmid, or a phage such as λ or M13 which is covalently linked to the vector DNA.

In general the method of this invention is initiated by linearizing the plasmid which contains a gene or DNA segment of interest (e.g., a phage resistance gene) and an ori region which is functional in the intended lactic acid bacterial host. The vector which contains a functional selectable phenotypic marker is also linearized. Linearization of the plasmid and the vector may be accomplished by techniques well-known to skilled molecular biologists (e.g., enzymatic digestion, mechanical shearing and the like). Advantageously, the plasmid and the vector are linearized by digestion with one or more restriction endonucleases which cleave the molecules at locations outside of: the ori region, at least one selectable marker, and the gene or DNA segment of interest. Often, one or more of the critical DNA regions contains a restriction site for a restriction enzyme used for linearization. In this case, the enzyme digestion is preferably conducted under partial digestion conditions, so as to ensure the formation of recombinant molecules with these regions intact.

The linearized vector and plasmid are combined under ligation conditions. Combination and ligation of the DNA may be accomplished by any of a variety of techniques, depending upon the cleavage procedure used. In general, complementary tailing, oligonucleotide linkers or digestion with similar restriction enzymes may be used to create cohesive ends that can be easily joined. Blunt ends may be created by certain restriction endonucleases or by filling in or exonucleolytic removal of staggered ends, then joined by blunt-end ligation techniques. Ligation is generally conducted in the presence of an appropriate DNA ligase, as is well-known in the art.

The resulting recombinant plasmids are then used to transform the lactic acid bacterial host. Transforming conditions involve contacting a solution of the recombinant plasmid with cells made competent to transformation. Lactic acid bacterial cells may be made competent to transformation, for example, by enzymatically removing their cell walls (i.e., preparing protoplasts) as taught by Kondo and McKay, Appl. Enviorn. Microbiol., 48:252-259 (1984) or by exposure of whole cells to a water-soluble lower polyalkylene glycol, as taught by Sanders et al., Appl. Environ. Microbiol., 53:1730-1736 (1987).

Transformants are cultivated on a selective medium and those which exhibit the phenotype encoded by the selectable marker are isolated for further evaluation. For example, if the selectable marker employed on the vector is erythromycin resistance, the transformants are selected by cultivation on an erythromycin-containing nutrient medium.

Often the desirable trait is not selectable and screening must be used. For example, phage resistance is screened by the presence or reduction of plaque formation.

The present method provides a reliable method for cloning DNA into lactic acid bacteria. The method is particularly useful for cloning genes which do not confer an easily selectable phenotype (e.g., phage resistance genes). The method employs the lactic acid bacterial origin of replication and a separate selectable marker such as antibiotic resistance, to provide for selection of transformants containing the desired genetic constructions.

There is also provided by the present invention a vector for cloning a plasmid-borne gene from a first lactic acid bacterium in a second transformable lactic acid bacteria. The vector is devoid of an origin of replication that is functional in lactic acid bacteria. The vector contains a phenotypic marker for selection in lactic acid bacteria and also advantageously contains restriction enzyme cleavage sites that permit cleavage of the vector and subsequent ligation in accordance with the method of this invention. The cleavage site chosen is either inherently compatible (as in sites generated by identical or compatible restriction enzymes or isoschizomer) or made to be compatible (by linker addition, filling-in reactions or exonucleolytic reactions) with a cleavage site on the plasmid containing the gene to be cloned, such that the two plasmids can be fused to form a recombinant plasmid containing the gene to be cloned, as well as a phenotypic marker for selection in lactic acid bacteria and an origin of replication that is functional in such bacteria.

In the following examples and the accompanying figures, the method of the invention will be described in detail with respect to the cloning, in S. lactis, of phage resistance gene derived from S. cremoris. Such cloning employs the following steps:

(1) A vector is prepared which contains an origin of replication for E. coli but not for lactic acid bacteria. The vector contains genes for chloramphenicol resistance (Cm$^r$), tetracycline resistance (Tet$^r$) and streptococcal erythromycin resistance (Erm$^r$). The vector further contains unique HindIII and EcoR1 restriction sites outside the Erm$^r$ gene.

(2) A plasmid containing a gene for a restriction-modification type phage resistance system is isolated from S. cremoris KH, by protoplast transformation of S. lactis LM0230 with S. cremoris KH plasmid DNA and selection of a colony that is resistant to LM0230-infecting bacteriophage.

(3) Isolated plasmids are partially digested with HindIII and ligated to HindIII-cut plasmid DNA from step 1.

(4) S. lactis LM0230 is transformed with the ligation mix and the transformants are grown on an erythromycin-containing nutrient medium. Selected transformants are further screened on phage-containing media. Transformants that are both erythromycin-resistant and phage-resistant contain the cloned gene.

In addition, the method of invention is described with respect to cloning a second type of phage resistance gene directly from a mixture of plasmids derived from a multiplasmid-containing strain of S. cremoris. Such cloning steps (1) of the previous description and the following steps:

6

(1) A plasmid pool containing undefined but presumed genes of interest is isolated from S. cremoris D11.

(2) Isolated plasmids are partially digested with EcoRI and ligated to EcoRI-cut vector DNA.

(3) S. lactis LM0230 is transformed with the ligation mix. Transformants are grown and selected as previously desribed.

The method of the invention provided an efficient method to identify and isolate plasmids containing a functional gene segment capable of conferring a desirable phenotype to a host lactic acid bacteria, in this case phage-resistance.

The application of cloned genes to the improvement of functional starter cultures is described with respect to the introduction of a recombinant plasmid containing phage resistance genes cloned by the method of this application to a metabolically proficient starter strain. Such strain improvment is described in the following steps:

(1) A recombinant plasmid containing pSA34 as a vector and a cloned phage resistance gene is prepared from a previous E. coli or streptococcal host.

(2) The plasmid is transformed into a metabolically proficient starter culture strain, and is shown to confer enhanced phage resistance.

The following examples further illustrate the invention, but are not to be construed as limiting.


## Example 1


Isolation of Bacteriophage Resistance Gene from S. cremoris KH and Cloning into S. lactis LM0230


Bacterial strains, phages and plasmids

The bacterial strains and phages employed are listed in Table 1 and plasmids are described in Table 2. The designation "pME100" refers to the 17.5-Kb plasmid as isolated from S. cremoris KH. However, this designation was not retained once the plasmid was transformed into S. lactis LM0230 since distinct plasmid-linked phenotypes of transformants resulted. Lactic streptococci and their phages were cultivated as described by Terzaghi and Sandine (Appl. Environ. Microbiol., 29, 807-813 (1975)), in M17 with glucose substituted for lactose (M17-glc). Overlay agar contained 0.6% agar instead of 0.45%. Both phage titers and spot assays were used to evaluate the phage resistance of cultures. Spot assays provided a quick screen and entailed spotting 5 $\mu$l of an overnight culture onto a lawn containing 1 ml of $10^{-4}$ dilution of a phage suspension containing approximately 5 x $10^9$ pfu/ml of c2 phage in overlay and 0.05 ml 1M CaCl$_2$. Plaque counts provided more accurate measurements of efficiency of plating (EOP). Antibiotic concentrations used were: 10$\mu$g/ml erythromycin (Erm) for streptococci; 15$\mu$g/ml tetracycline (Tet) for E. coli; 100$\mu$g/ml chloramphenicol (Cm) for E. coli. E. coli cells were grown in L broth (Silhavy, et al., Experiments with Gene Fusions, Cold Spring Harbor Laboratory (1984)). $\lambda^{vir}$ titrations were conducted using TB agar as described by Silhavy, et al. (id).

## Table 1

| Strain/Plasmid | Homologous Phage | Plasmid Content | Bacterial Phenotype[a] | Ref/origin |
|---|---|---|---|---|
| S. lactis LM0230 | c2 | none | R/M$^-$ | McKay, et al.[c] |
| S. cremoris KH | kh, c2[b] | Multi | R/M$^+$ | Sanders & Klaenhammer[d] |
| S. cremoris M49 | kh, c2[b] | KH cured of pME100 | R/M$^-$ | Sanders & Klaenhammer[d] |
| E. coli DH5 | λvir | none | | Bethesda Research Labs |

[a] R/M- host controlled restriction/modification system

[b] c2 phage replicates on KH with an EOP of $10^{-7}$ and M49 with an EOP of $10^{-4}$ (Sanders & Klaenhammer).

[c] Appl. Environ. Microbiol., 47: 68-74 (1983)

[d] Appl. Environ. Microbiol., 42: 944-950 (1981)

## Table 2

| Plasmid | Size (kb) | Phenotype[a] | | Description | Origin |
|---|---|---|---|---|---|
| | | Strep | E. coli | | |
| pME34 pME35 pME36 pME37 | 23.5 | R/M$^+$ Erm$^+$ ori+ | Cm$^r$ Tet$^S$ R/M$^-$ ori$^+$ | Plasmids represent different arrangements pME1a + psA34 DNA | This example |
| pHP2 | 23.5 | R/M$^-$ Erm$^+$ ori$^+$ | Cm$^r$ Tet$^S$ R/M$^-$ ori$^+$ | pME1a + pSA34 | This example |
| pME1a | 17.5 | R/M$^+$ ori$^+$ | – | Naturally occurring plasmid from S. cremoris KH | This example |
| pSA3 | 10.3 | Erm$^+$ ori$^+$ | Cm$^r$ Tet$^r$ ori+ | E. coli/ Streptococcal shuttle vector | Dao and Ferretti[b] |
| pSA34 | 6.0 | erm$^+$ ori$^-$ | Cm$^r$ Tet$^r$ ori+ | HindIII cutback of pSA3 with in vivo deletion upstream from erm gene | This example |
| pME100 | 17.5 | R/M$^+$ ori$^+$ | – | Indigenous plasmid preparation from S. cremoris KH. | Sanders and Klaenhammer[c] |

[a] R/M – restriction/modification system; Tet$^r$ – Tetracycline resistance; Cm$^r$ – chloramphenicol resistance; Erm$^r$ – erythromycin resistance; ori – origin of replication region.

[b] Appl. Environ. Microbiol., 49:115-119 (1985)

[c] Appl. Environ. microbiol., 42:944-050 (1981)

### Plasmid DNA isolation

Plasmid DNA was isolated from streptococci according to Anderson and McKay (Appl Environ. Microbiol., 46, 549-552 (1983)), with modifications as described by Sanders and Nicholson (Appl. Environ. Microbiol., 53:1730-1736 (1987)) to render DNA digestable with restriction enzymes without a cesium chloride (CsCl) gradient purification. For CsCl-gradient purification of streptococcal plasmids, the procedure of Klaenhammer, et al., Appl Environ. Microbiol., 35:592-600 (1978), was followed, except cells were grown in M17 from Difco Labs, a broth containing 5g/L glucose instead of lactose designated M17-glc, and lysozyme treatment was extended to 30 min. E. coli plasmid DNA was isolated according to the alkaline extraction method described by Silhavy, et al. (supra). For CsCl-gradient centrifugation, the procedure was scaled-up for 1L. of cells, and ethanol precipitated DNA was brought to a refractive index of 1.3970 with solid CsCl or TE buffer (10mM tris, 1mM EDTA, pH 8.0). Ethidium bromide was added to a final concentration of 0.25 mg/ml. Gradients were centrifuged 47000 rpm in a Beckman L8-55 (Palo Alto, California) ultracentrifuge using a Ti60 rotor. Ethidium bromide was removed from DNA by extraction with CsCl-saturated isopropanol, DNA was dialyzed overnight in TE buffer, and ethanol precipatated. pME100 was separated from other plasmids in S. cremoris KH by cutting the desired band from a low melting point agarose (LMP, Bethesda Research Labs, Gaithersburg, MD) gel. Approximately 50-100 μg DNA was loaded into a preparative well of a 20 x 15 cm gel. After electrophoresis, a band of approximately 1 cm in width of the gel was cut from the edge and stained to localize the position of the desired band. The band was excised, melted at 70°C, and diluted with an equal volume of TE buffer. Phenol saturated with 20X TE was used to extract the gel/DNA mixture at room temperature. Single extractions with phenol:chloroform (1:1), and chloroform:isoamylalcohol (24:1) followed. The aqueous phase was precipitated with 2 volumes 95% ethanol and 0.1 volume 3M sodium acetate at -20°C/overnight. DNA was recovered by centrifugation at 15,000 x g for 30 min. DNA was resuspended in approximtely 80 μl TE buffer.

Restriction and modification assays

Both restriction and modification phenotypes were assayed solely by in vivo techniques. Restriction was defined by the decrease of phage plaquing capability relative to a non-restricting host. Modification was defined as the restoration of full level phage plaquing after one cycle of growth through the restricting host. To determine restrictive capcity, phage were titrated according to Terzaghi and Sandine, Appl. Environ. Microbiol., 29, 807-813 (1975), on the fully permissive (non-restricting) host and on the restricting host. Efficiency of plating (EOP) was calculated as: titer on restrictive host divided by titer on permissive host. To determine modification, single plaques emerging at low EOP on restrictive hosts were picked with a sterile 1 ml pipet into 1 ml M17-glc broth, vortexed and plated on both restrictive and permissive hosts. Modification was assumed when phage titers on restrictive hosts were equal to phage titers on non-restrictive hosts.

DNA manipulations

DNA restriction digests and ligations were performed according to enzyme manufacturers' instructions. Enzymes were purchased from New England Biolabs (Beverly, MA) or Boehringer Mannheim (Indianapolis, IN). Cloning procedures were followed as described by Maniatis, et al., Molecular Cloning: A Laboratory Manual, Cold Spring Harbor Laboratory (1982).

Transformation

Transformation of S. lactis LM0230 was conducted using two procedures. For transformation of LM0230 with pME100, the protoplast transformation procedure of Kondo and McKay (Appl. Environ. Microbiol., 48: 252-259, (1984)) was used except mutanolysin (Miles Scientific, Elkhart, IN) concentration was decreased to 0.25 μg/ml. Regeneration was also conducted on the medium described by Okamoto et al., Agric. Biol. Chem., 47: 259-263 (1983). Subsequent transformations of ligation mixes into LM0230 were conducted by the whole-cell transformtion procedure of Sanders and Nicholson (Appl. Environ. Microbiol., 53:1730-1736 (1987)). E. coli DH5 competent cells were obtained from a commercial source and were transformed as suggested by the manufacturer.

Isolation of pME100 from S cremoris KH and transformation into LM0230

The plasmid pME100 was isolated from the multi-plasmid-containing S. cremoris KH background as described above. This DNA was used to protoplast transform S. lactis LM0230. Transformants were directly selected on the basis of resistance to c2 phage. Of 26 phage-reisistant colonies isolated after transformation, 10 were found, by agarose gel electrophoresis to contain a 17.5-Kb plasmid. Phage-resistant colonies not containing the plasmid were presumably phage-resistant mutants. Each transformant was streaked to purify it from contaminating phage and four isolates of each were characterized for phenotype and plasmid content.

Phenotype of the pME1a transformant

One transformant, designated 1a, contained the 17.5 kb plasmid and exhibited restriction modification activity. The EOP of c2 phage on 1a was $8.7 \times 10^{-5}$. This restriction was reversed by one cycle of growth through the modifying host, 1a (Table 3). To verify that the phage resistance phenotype of 1a was due to the transformed plasmid, named pME1a for convenience, and not due to mutation, a curing study was conducted. After multiple transfers under non-selective conditions 11 single colony isolates of 1a were tested for phage resistance and plasmid content. All phage resistance isolates (total of 6) retained pME1a, whereas all phage sensitive isolates (total of 5) were cured of pME1a. Therefore, the phage resistance phenotype correlated with the presence of pME1a, confirming the linkage of this trait to the plasmid.

## Table 3
### Host-Controlled Modification of c2 phage Grown Through LM0230 and 1a

| Phage | | Host | pfu/ml | log EOP |
|---|---|---|---|---|
| c2 | x | LM0230 | $2.3 \times 10^{10}$ | 0 |
| | | 1a | $2.9 \times 10^{6}$ | $-4.06$ |
| mc2.1a | x | LM0230 | $2.2 \times 10^{7}$ | 0 |
| | | 1a | $1.5 \times 10^{7}$ | $- .17$ |
| mc2.1a.LM0230 | x | LM0230 | $1.1 \times 10^{6}$ | 0 |
| | | 1a | $2 \times 10^{3}$ | $-2.4$ |
| mc2.LM0230 | x | LM0230 | $8.0 \times 10^{7}$ | 0 |
| | | 1a | $3.8 \times 10^{5}$ | $-2.35$ |

To determine phage resistance characteristics conferred by pME1a against a variety of LM0230-infecting phages, 1a was challenged with nine additional independently isolated phages. Eight of these phages were prolate-headed and one isometric-headed. The results showed a decreased EOP of $7.9 \times 10^{-2}$ to $4.3 \times 10^{-6}$ characterizing the pME1a phage resistance. All phages were also host-modified after one cycle of growth through the transformant. These results indicated that this phage-resistance mechanism was active against typical phage isolated from dairy fermentations. Plasmid pME1a, in a S. lactis host has been deposited with the American Type Culture Collection, with accession number ATCC 67553.

Cloning of pME1a

To determine useful cloning sites in pME1a, this plasmid was digested with several enzymes, and the number of sites and approximate size of fragments were determined (Table 4). Of 18 enzymes screened, none cut pME1a only once.

11

## Table 4

| Enzyme | # sites | Site (kb) of fragment | kb Total |
|--------|---------|------------------------|----------|
| Pvu I | 2 | 13.8, 4.17 | 17.97 |
| Pvu II | 3 | 15.14, 1.29, 1.05 | 17.48 |
| Xba I | 4 | 11.30, 4.47, 1.58, 0.70 | 18.05 |
| Acc I | 5 | 5.35, 3.87, 3.87, 2.09, 2.01 | 17.09 |
| Ava I | 3 | 15.0, 1.36, 0.5 | 16.86 |
| Ava II | 4 | 7.59, 4.12, 3.76, 1.78 | 17.25 |
| Bcl I | 4 | 10.0, 3.24, 2.99, ~0.8 | 17.03 |
| Cla I | 3 | 12.16, 4.87, 0.95 | 17.98 |
| Eco RV | 6 | 7.08, 3.27, 3.13, 1.66, 1.51, 1.24 | 17.89 |
| Hind III | 3 | 11.75, 5.43, 1.30 | 18.48 |
| Nde I | 2 | 13.80, 3.89 | 17.69 |

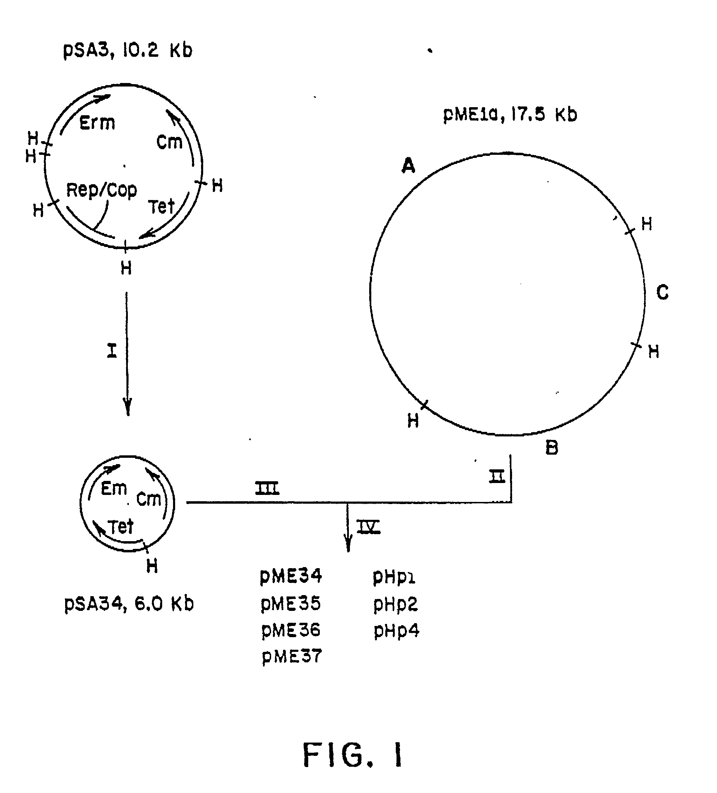

The strategy used to clone pME1a is shown in Fig. 1. A vector void of an origin of replication for lactic acid bacteria was constructed from pSA3 (Dao & Ferretti, Appl. Environ. Microbiol., 49:115-119 (1985)). Plasmid pSA3 (5 μg) was digested with HindIII and religated in the presence of T4 DNA ligase. A 6.0-kb plasmid was isolated from E. coli transformants of the religation mixture. This vector, pSA34, is composed of the two largest HindIII fragments from pSA3 minus approximately 1Kb of DNA spontaneously deleted in vivo. pSA34 has a unique HindIII site in the Tet$^r$ gene promoter, carries Tet$^r$ and Cm$^r$ and an origin of replication for selection in E. coli and Erm$^r$ for selection in Streptococcus. This vector allows direct selection of a cloned streptococcal plasmid in S. lactis since it lacks an origin of replication for streptococci. Only vectors providing Erm$^r$, with cloned insert DNA providing an ori region functional in streptococci, will produce colonies with selection on erythromycin. The plasmid pSA34 in an E. coli host, has been deposited at the American Type Culture Collection with accession number ATCC 67552.

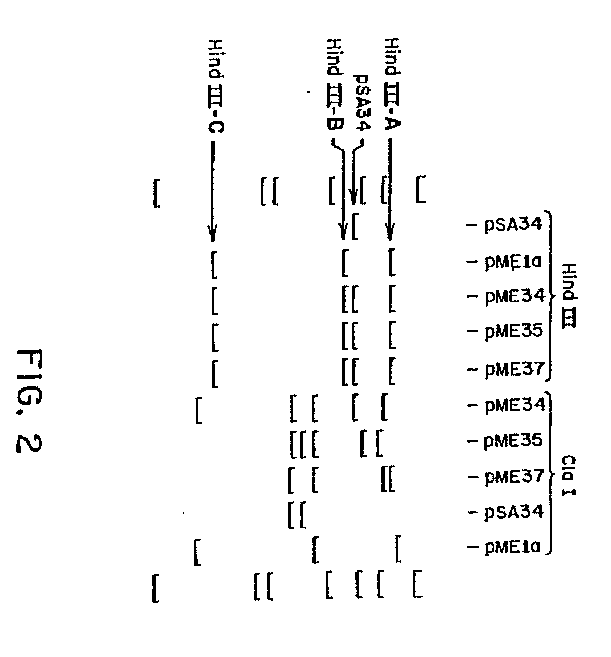

pME1a DNA (5 μg) was randomly linearized by HindIII at 1 of the 3 HindIII sites present on pME1a by partial digeston in the presence of ethidium bromide (20 μg/ml). This DNA was ligated to HindIII-digested pSA34, (5 μg) and the ligation mix was used to transform S. lactis LM0230. All Erm$^r$ transformants were screened for pME1a-type phage resistance to c2 phage. Of 47 Erm$^r$ transformants, 4 were c2 phage resistant. These clones were designated pME34, pME35, pME36 and pME37. Plasmid analysis revealed that all three HindIII fragments of pME1a were cloned in each of the isolates. Figure 2 shows a partial restriction map of pME34 and the location of HindIII fragments A, B and C from pME1a. However, digests by alternative enzymes demonstrated that pME34 and pME36 were identical, but distinct from pME35 and pME37. The plasmid, pME34 in an S. lactis (LM0230) host has been deposited at the American Type Culture Collection with accession number ATCC 67554. Several phage-sensitive clones also contained all three pME1a HindIII fragments. Restriction analysis of these clones showed that insertion at either the HindIII sites directly flanking the HindIII C fragment (see Fig. 1) resulted in insertional inactivation of the phage-resistance genes.

## Table 5

| Titrating Host | pfu/ml | EOP |
|---|---|---|
| LM0230/pME34 | $4.7 \times 10^6$ | $1.9 \times 10^{-4}$ |
| LM0230/pME35 | $6.2 \times 10^6$ | $2.5 \times 10^{-4}$ |
| LM0230/pME36 | $3.6 \times 10^6$ | $1.4 \times 10^{-4}$ |
| LM0230/pME37 | $1.6 \times 10^7$ | $6.4 \times 10^{-4}$ |
| LM0230 | $2.5 \times 10^{10}$ | $1$ |
| LM0230/pHP2 | $2.1 \times 10^{10}$ | $8.8 \times 10^{-1}$ |

Phenotype of pME1a clones

Each of the phage-resistant clones was assayed for its ability to confer phage resistance on S. lactis LM0230 (Table 5). Phage was restricted from 3.2 to 3.9 log cycles when grown in S. lactis LM0230 containing the cloned DNA. Several isolates (typified by pHP2) contained all pME1a DNA but did not restrict phage developMent. Each plasmid was then transformed into E. coli DH5 selecting for Cm$^r$. Phage-resistance was not expressed in the E. coli host as determined by plaquing efficiencies of $\lambda^{vir}$ phage. It is unknown if this result is due to the failure of the gene product to be produced in adequate quantity to produce a phage-resistance phenotype in E. coli or an inactivity of these proteins against $\lambda^{vir}$. All pME34, pME35, pME36 and pME37 transformants of E. coli were Tet$^s$ since cloning into the HindIII site insertionally inactivates this marker.

Restriction mapping and localization of phage-resistance genes of pME34

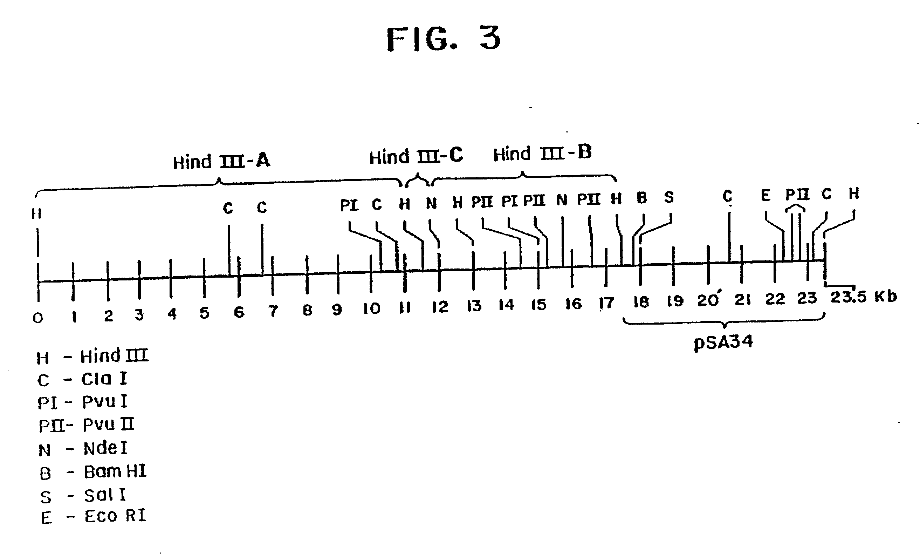

Using multiple digestions of whole or gel isolated fragments of pME34 the restriction map shown in Fig 3 was deduced. The DNA was mapped relative to PvuI, PvuII, ClaI, SalI, BamHI, EcoRI, and NdeI. The phage-resistance genes on pME34 were localized using deletion and insertion mapping. It was found that deletion of either the internal PvuI fragment, or both PvuII fragments resulted in phage-sensitivity. Furthermore, insertion of pSA34 at either HindIII site flanking the HindIII-C fragment resulted in insertional inactivation of phage resistance genes. Therefore, the phage-resistance genes appear to reside between map positions 11 and 15 as shown in Fig. 3.

## Example 2

Bacterial Strains, Phages and Plasmids

Strain S. cremoris D11 is a mesophilic starter culture commercially available from Marschall Products. This strain naturally harbors five plasmid species. pSA34 is as previously described. Strain LM0230 was used as a transformable host for cloning reactions. Phages c2, sk and p2 were used as homologous phage for LM0230 and LM0230 containing cloned plasmid DNA. Microbiological manipulations are as described in Example 1.

Cloning of plasmid DNA from S. cremoris D11

Total plasmid DNA was extracted from S. cremoris D11 as described previously. Approximately 10μg of plasmid DNA was digested with 50U EcoRI enzyme for 20 min at 37°C. This resulted in fragmentation of the four largest plasmids. This DNA was extracted with 1:1 50mM Tris saturated phenol: chloroform and ethanol precipitated. pSA34 vector DNA was digested to completion with EcoRI, extracted and ethanol precipitated as described for D11 plasmid DNA. Ligations were performed using 400U DNA ligase and approximately 5μg each D11 plasmid DNA and pSA34 DNA. Ligation mixes were transformed directly intoLM0230 using the non-protoplast transformation system. Seven erm$^r$ transformants were recovered from D11 plasmid cloned into pSA34, designated PD11A → PD11G.

Phenotype of pD11 clones

All seven pD11 clones (A→G) were sensitive to c2 phage (prolate-headed). However, when tested against isometric-headed phages sk and p2, clone pD11A showed increased resistance. The EOP of p2 phage on pD11A was $10^{-5}$ and sk phage was $10^{-4}$. Therefore, the gene segment from S. cremoris D11 cloned onto pSA34 confers enhanced resistance to some LM0230 phages.

This example demonstrates the utility of pSA34 for cloning uncharacterized plasmids from lactic acid bacteria. In this case, individual plasmid DNA's were not purified and there was no prior biological evidence that specific plasmids encoded any useful or interesting phenotypes. The only restriction to the application is that the DNA to be cloned must be associated with an origin of replication region in the transformed lactic acid bacterium.

Example 6

Once useful genes are identified by the cloning method described, these genes can be used to improve starter cultures. The transformable host (LM0230) used for cloning experiments is not capable of metabolizing lactose or digesting protein.

To prepare a metabolically proficient starter culture with improved phage resistance, pME34 was transformed into a Lac$^+$Prt$^+$ strain, S. lactis C2. Erm$^r$ transformants were shown to contain pME34 by agarose gel electrophoresis. The acid-producing activity of S. lactis C2 containing pME34 (C2/pME34) was determined in the presence and absence of phages. sk, p2, c2 and cc7 phages were added at levels of 180, 150, 330 and 35 pfu, respectively, to 10 ml of 60 min steamed milk containing 2% (v/v) of overnight cultures of C2, as a control, or C2/pME34. After 8 hour growth at 30°C, the pH of the culture milk was determined. As can be seen from the data in Table 6, C2/pME34 exhibited enhanced resistance to all phages tested and retained the acid-producing activity in milk as seen in the parent strain.

## Table 6
### Acid Development of C2/pME34 and C2
### With and Without Phage Challenge

| Phage | Host | |
|---|---|---|
| | C2/pME34 | C2 |
| sk | 4.89 | 6.20 |
| p2 | 4.91 | 6.28 |
| c2 | 4.91 | 6.23 |
| cc7 | 4.94 | 6.21 |
| no phage | 4.84 | 4.87 |

The increased phage resistance of C2/pME34 was also demonstrated by phage titration. The EOP of sk, p2, c2 and cc7 phages on C2/pME34 was $2.4 \times 10^{-5}$, $3.0 \times 10^{-5}$, $6.9 \times 10^{-5}$ and $1.6 \times 10^{-5}$, respectively, as compared to titers on C2.

Therefore, the phage resistance of the metabolically functional starter culture, C2, was enhanced by the introduction of a recombinant plasmid, pME34, derived by the methodology previously described. Both C2 and C2/pME34 demonstrate acid-producing activity in milk, yet C2/pME34 is superior to C2 in the presence of the 4 phages tested. This is the first example of a functional starter culture improved by in vitro

14

recombinant DNA technology.

Many modifications and variations can be made without departing from the spirit and scope of the invention which is solely defined by the claims.

## Claims

1. A method for producing a transformed lactic acid bacterium from a lactic acid bacterial host and isolating said transformed lactic acid bacterium, wherein the transformed bacterium contains a plasmid-borne functional gene segment capable of conferring a phenotype not previously present in the lactic acid bacterial host, the method comprising the steps of:

(a) forming a recombinant plasmid by joining, under DNA-ligating conditions, (i) a first plasmid from a lactic acid bacterium, said plasmid having a gene of interest and an origin of replication which is functional in the lactic acid bacterial host with (ii) a vector which contains a selectable marker but does not contain an origin of replication which is functional in the lactic acid bacterial host;

(b) directly transforming the lactic acid bacterial host with the recombinant plasmid under transforming conditions;

(c) cultivating the transformants resulting from step (b) and selecting and isolating a transformant which exhibits the phenotype conferred to the host by the selectable marker.

2. The method of claim 1 in which the plasmid containing lactic acid bacterium and the host lactic acid bacterium are both lactic streptococci.

3. The method of any of claims 1 and 2 in which the host streptococcus lactic acid bacterium is S. lactis.

4. Use of a streptococcus lactic acid bacterium produced by the method of any of claims 1 to 3 as a starter culture bacterium.

5. A vector comprising a covalently closed recombinant plasmid deficient in an origin of replication for lactic acid bacterium but containing a marker selectable in lactic acid bacterium, preferably an antibiotic resistance marker selectable in lactic acid bacterium.

6. The vector of claim 5 additionally comprising an origin of replication for a secondary host cell and optionally a restriction enzyme cleavage site.

7. The vector of claim 6 which is identified as pSA34.

8. A transformant obtained by the method of claim 1, comprising; a lactic acid bacterium host cell containing a recombinant plasmid, which plasmid is composed of:

(a) a marker selectable in the host;

(b) a gene encoding a gene product not previously produced by the host cell, preferably the gene product results in a phenotype, such as phage resistance, not previously observed in the host; and

(c) an origin of replication functional in the host cell; and optionally additionally comprising an origin of replication functional in a secondary host.

9. A method for identifying a functional gene segment capable of conferring a phenotype to a lactic acid bacterium not previously exhibiting such phenotype, comprising the steps of:

(a) combining a plasmid from a first lactic acid bacterium with a vector identified as pSA34, the lactic acid bacterium plasmid including a gene capable of conferring a phenotype not previously exhibited by a host lactic acid bacterium and an origin of replication functional in the host;

(b) transforming, directly, the recombinant plasmid into the lactic acid bacterium host to form a transformant;

(c) culturing the transformant on an agar containing erythromycin; and

d) screening the cultured transformant for the phenotype;

wherein the phenotype is preferably phage resistance and the screening is for the absence or reduction of plaque formation.

10. A starter culture, comprising: a lactic acid bacterium which has the ability to ferment milk, containing the recombinant plasmid obtained by the method of any of claims 1 ot 3 or a function gene segment of interest found in the recombinant plasmid.

15

FIG. I

FIG. 2

# FIG. 3

Hind III-A    Hind III-C    Hind III-B

H — Hind III
C — Cla I
PI — Pvu I
PII — Pvu II
N — Nde I
B — Bam HI
S — Sal I
E — Eco RI

pSA34

EP 0 316 677 A2